# EUROPEAN PATENT SPECIFICATION

(11) **EP 2 927 230 B1**
(45) Date of publication and mention of the grant of the patent: **23.08.2017**
(21) Application number: 15162292.5
(22) Date of filing: 01.04.2015
(51) Int. Cl.: C07D 417/14

(54) **PENTAMETHINIUM SALTS WITH AN EXPANDED QUINOXALINE UNIT AND ITS USE IN ANTICANCER THERAPY**
PENTAMETHINIUMSALZE MIT EINEM ERWEITERTEN CHINOXALIN UND DESSEN VERWENDUNG IN DER ANTIKREBSTHERAPIE
SELS DE PENTAMÉTHINIUM AVEC UNE UNITÉ DE QUINOXALINE EXPANSÉE ET SON UTILISATION EN THÉRAPIE ANTICANCÉREUSE

(30) Priority: 01.04.2014 CZ 20140213
(43) Date of publication of application: 07.10.2015
(73) Proprietor: University of Chemistry and Technology - Prague, 166 28 Prague 6 (CZ); First Faculty of Medicine, 121 08 Prague 2 (CZ); Institute of Molecular Genetics of ASCR, v.v.i., 142 20 Prague 4 (CZ)
(72) Inventor: Kral, Vladimir, 120 00 Prague 2 (CZ); Havlik, Martin, 163 00 Prague 6 (CZ); Kaplanek, Robert, 102 00 Prague 10 (CZ); Dolensky, Bohumil, 273 03 Stochov (CZ); Ruml, Tomas, 100 00 Prague 10 (CZ); Rimpelova, Silvie, 472 01 Doksy (CZ); Martasek, Pavel, 148 00 Prague 4 (CZ); Briza, Tomas, 148 00 Prague 4 (CZ); Kejik, Zdenek, 356 01 Sokolov (CZ); Rak, Jakub, 323 00 Plzen (CZ); Kralova, Jarmila, 120 00 Prague 2 (CZ)
(74) Representative: Beetz & Partner mbB

(56) References cited:
- AVIRAH, R.R.: "Squaraine dyes in PDT: from basic design to in vivo demonstration", ORGANIC & BIOMOLECULAR CHEMISTRY, vol. 10, 3 November 2011 (2011-11-03), pages 911-920, XP002740695,
- RAPOZZI, V. ET AL.: "Photooxidation and Phototoxicity of pi-Extended Squaraines", J. MED. CHEM., vol. 53, 2 April 2010 (2010-04-02), pages 2188-2196, XP002740696,
- BRIZA, T. ET AL.: "Striking Antitumor Activity of a Methinium System with Incorporated Quinoxaline Unit Obtained by Spontaneous Cyclization", CHEMBIOCHEM, vol. 16, 28 January 2015 (2015-01-28), pages 555-558, XP002740698,

## Description

### Abstract

The invention describes the use of systems based on pentamethinium salts with an expanded quinoxaline unit, the unit being incorporated into a pentamethinium chain. The systems can be used in anticancer therapy. The systems are based on the use of a structural motif of pentamethinium salts prepared from corresponding malondialdehyde with a quinaxoline unit.

### State of the art

Quinoxaline derivatives are wide-spread in nature and some of them, as for example the antibiotic, levomycin show significant biological activity. Many synthetic quinaxolines have been prepared and some of them were used as antibacterial agents, antidepressants, insecticides, fungicides, whereas the other quinoxaline derivatives are tested as compounds with significant biological activity. *(*G.W.H.Cheeseman, Adv. Heterocycl. Chem. 2, 203 (1963*),* Kim, Y. B., Kim, Y. H., Park, J. Y., Kim, S. K. (2004): Synthesis and biological activity of new quinoxaline antibiotics of echinomycin analogues, Bioorg. Med. Chem. Lett. 14, 541-544*.)*

Undoubtedly, the quinoxaline unit is responsible for biological activity, which can be positively modified by connecting it with other biologically and analytically interesting units. Such units can be, for example, polymethinium salts with a wide-spread utilization. Mishra, A.; Behera, R. K.; Behera, P. K.; Mishra, B. K.; Behera, G. B. (2000) Cyanines during the 1990s: a review. Chem. Rev. 100, 1973-2012*.* In our laboratory, we have prepared various structures based on methinium salts, showing versatile use in the field of modern chemistry. We have prepared biologically significant binaphthyl derivatives with methinium substitution Briza, T., Kejík, Z., Vašek, P., Králová, J., Martásek, P., Císarová, I. and Král, V. (2005): Chromophoric binaphthyl derivatives. Organic Letters. 7, 3661-3664*.),* pentamethinium systems behaving as analytical sensors for heparin and sulfated biopolymers Bříza, T., Kejík, Z., Císařová, I, Králová, J., Martásek, P., Král, V. (2008): Optical sensing of sulphate by polymethinium salt receptors: Colorimetric sensor for heparin. Chemical Communication. 1901-1903*.,* further derivatives of porphyrine with methinium substitution Bříza, T.; Králová, J.; Cígler, P.; Kejík, Z.; Poučková, P.; Vašek, P.; Moserová, I.; Martásek, P.; Král, V. Combination of Two Chromophores: Synthesis and PDT application of Porphyrin-Pentamethinium conjugate: Bioorganic & Medicinal Chemistry Letters, Volume 22, Issue 1, 1 January 2012, Pages 82-84*.)* and pentamethinium salts which can be used as selective mitochondrial probes *(*Rimpelova S., Briza T., Kralova J., Zaruba K., Kejik Z., Cisarova I., Martasek P., Ruml T. and Kral V. 2013. Rational design of chemical ligands for selective subcellular targeting. Biocon. Chem., 24: 1445-1454*,* Briza T., Rimpelova S., Kralova J., Zaruba K., Kejik Z., Ruml T., Martasek P., Kral V. 2013. Pentamethinium fluorescent probes: the impact of molecular structure on photophysical properties and subcellular localization. Dyes Pigments. published online; DOI 10.1016/j. dyepig. 2013.12.021*).*

The combination of quinoxaline and a methinium chromophoric system offers a substance with better biological and optical properties, or the resulting substance will have novel unique properties. For example, the connection of the quinoxaline derivative with a methinium system enhances an easy detection on a cellular level.

Some examples of a linkage of the quinoxaline unit and a methinium system were *described (*Cook, A. H., Garner, J., Perry, C. A. (1942): Quinoxaline cyanines. Part I; 710-713*.;* Cook, J., Perry, C. A. (1943): Quinoxaline cyanines. Part II; 394-397*;* Abu El-Hamd, R. M., Koraiem, A. I. M. (1990): Studies on the synthesis of conjugated five-six biheterocyclic cyanine dye series. J. Islamic Academy of Science, 3:4, 262-268*;* US Patent No. 3632808, 1972*;* US Patent 4356244, 1982*.)* It concerns substances where the quinoxaline unit is connected to the side end of heteroaromates of a methinium chain. The biological properties of these compounds have not been studied. The compounds were characterized and their optical properties have been described. In different works, methinium salts with a quinoxaline unit were described as antagonists of neurokinine receptors. *(*Appell, K. C., Babb, B. E., Goswami, R., Hall, P. L., Lawrence B. K., Logan, M. E., Przyklek-Elling, R., Tomczuk, B. E., Venepalli, B. R., Yanni, J. M. (1991): Imidazo[4,5-b]quinoxaline Cyanines as Neurokinin Antagonists, J. Med. Chem. 34, 1751-1753*).*

### The gist of the invention

This pentamethinium system with an incorporated expanded quinoxaline unit is unique because no such system was prepared up to date. The compound enhances very interesting cytotoxic properties on selected cellular lines *in vitro* and shows a significant suppression of cancer growth on the living nu/nu-mouse model.

The system is based on pentamethinium salts with an expanded quinoxaline unit, which is incorporated into a pentamethinium chain of general formula I, 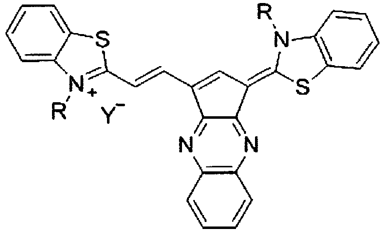 Compound of general formula I
wherein both end-side heteroaromatic groups are the same and are formed by a substituted benzothiazol, their structures being characterized by group R and Y, where group R is an alkyl chain of a length of C1 to C8, and Y is chloride, bromide or iodide.

'Alkyl chain' having a length of C1 to C8, refers to a hydrocarbon chain with 1 to 8 carbon atoms in length. Such hydrocarbon chains are preferably but not necessarily saturated and may be branched or straight chain, although typically straight chain is preferred. Exemplary alkyl groups include methyl, ethyl, propyl, isopropyl, butyl, isobutyl, sec-butyl, tert-butyl, pentyl, 3-methylpentyl, and the like. Preferred alkyl chains are propyl and isopropyl.

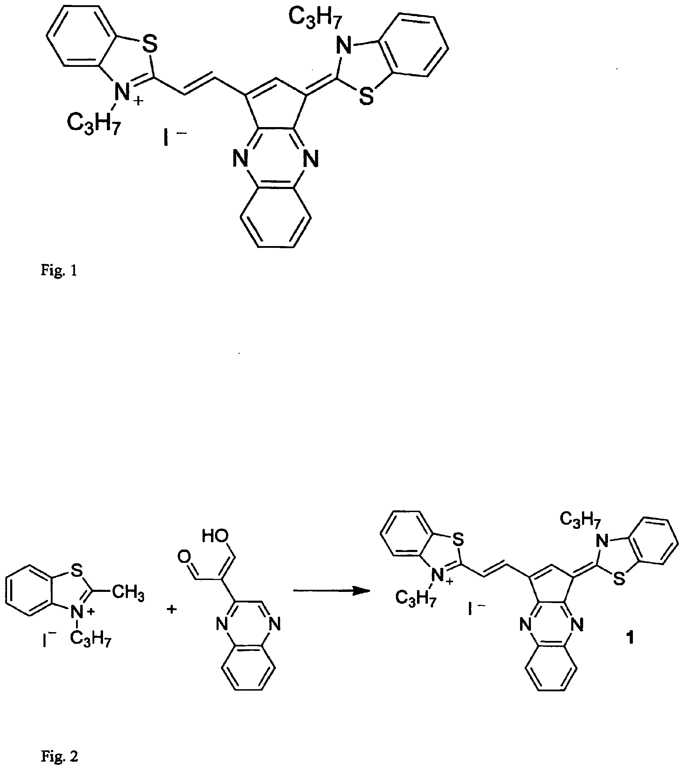

Our structural motif, in comparison with known quinoxaline-methinium salt derivatives, is absolutely unique and novel. The pentamethinium system with an expanded quinoxaline unit incorporated into a pentamethinium chain is introduced in the following Figure (Fig. 1).

The object of the invention are pentamethinium systems with incorporated expanded quinoxaline units of general formula I for the preparation of an agent with significant cytotoxic properties which can be used for cancer treatment.

We surprisingly observed that compound **1** displayed high cytotoxicity for the following human tumor lines MCF-7 (breast cancer cells), MDA-MB-231 (human breast carcinoma), U-2 OS (human osteosarcoma), PE/CA-PJ34 (human basaloid squamous cell carcinoma), MiaPaCa-2 (human pancreatic carcinoma), LNCaP (human prostate carcinoma) a 4T1(mouse mammary gland carcinoma).

The *in vivo* study realised on the Nu/nu mice model with human breast carcinoma (MDA-MB-231) showed high anticancer effect of compound **1**. After its application, the tumor growth was practically stopped (Figure 2) for the duration of the experiment (more than 40 days).

Pentamethinium salts with an incorpoarted expanded quinoxaline unit, which are the object of this patent, are based on the condensation of a corresponding malondialdehyde with a quinoxaline unit and a corresponding heteroaromatic salt (Scheme 1) by a method which is described for similar systems *(*Bříza, T., Kejík, Z., Císařová, I., Králová, J., Martásek, P., Král, V. (2008): Optical sensing of sulphate by polymethinium salt receptors: Colorimetric sensor for heparin. Chemical Communication. 1901-1903*).*

### Summary of the Figures

- Fig. 1:: Pentamethinium system with an expanded quinoxaline unit incorporated into a pentamethinium chain,
- Fig. 2:: Preparation of pentamethinium salts with an incorpoarted expanded quinoxaline unit, according to Example 1.

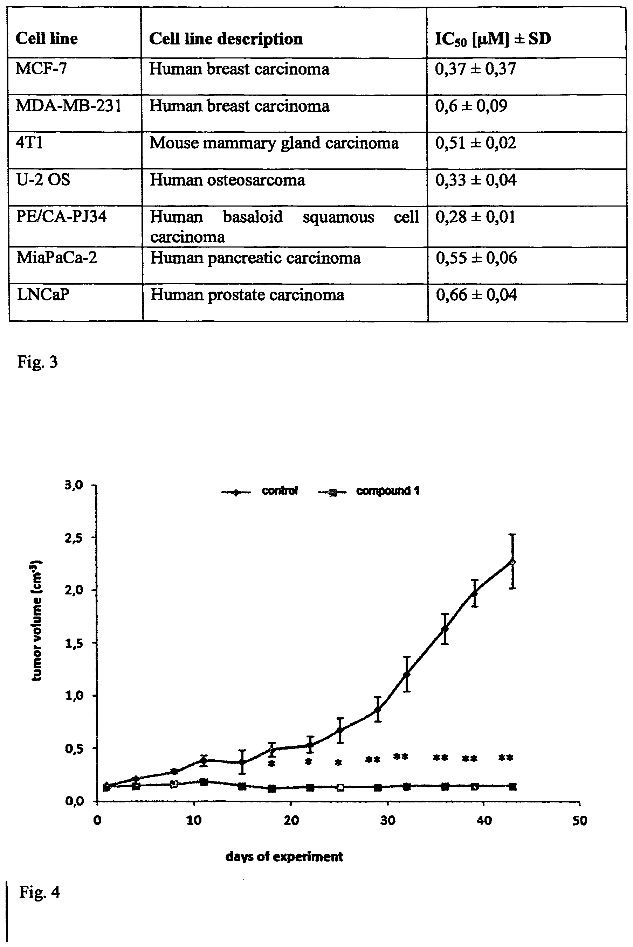

- Fig. 3:: Cytotoxic activity of compound 1 expressed by IC50 (after 72 h) malignant and non-malignant (BJ) cell lines, according to Example 2.
- Fig. 4:: Effect of salt 1 on tumor growth in vivo. Differences in the tumor volume of control mice versus mice treated with compound 1, according to Example 3.

### Examples demonstrating the preparation and properties of compound 1

### Example 1

Preparation of compound **1**. The mixture of 2-quinoxaline malondialdehyde (50 mg), 2-metyl-3-propyl benzothiazole (180 mg) and dry n-butanol (7 mL) was heated to 110 °C for 18 hours. After cooling to laboratory temperature, the product was separated by filtration, washed several times with methanol (3 mL) and dried in vacuum. The product was obtained as a dark green powder. Yield: 136 mg, 81%.

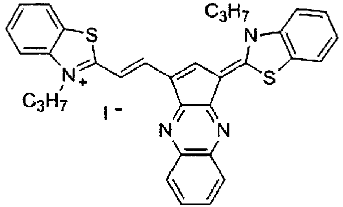

### Structure of compound 1

Characterization: **¹H-NMR** (500 MHz, DMSO-*d*₆, 25 °C: 8.65 (1H, br s), 8.49 (1H, br d, 14.5), 8.33 (1H, d, 14.5), 8.19 (1H, d, 7.8), 8.18 (1H, d, 7.7), 8.14 (1H, d, 8.3), 8.13 (1H, d, 8.1), 8.06 (1H, d, 8.1), 8.01 (1H, d, 8.2), 7.78 (1H, m), 7.75 (1H, m), 7.73 (1H, t), 7.66 (1H, t, 7.7), 7.61 (1H, t, 7.6), 7.47 (1H, t, 7.5), 4.78 (2H, t, 7.4), 4.65 (2H, t, 7.2), 2.06 (2H, m), 2.03 (2H, m), 1.22 (3H, t, 7.4), 1.20 (3H, t, 7.4); **¹³C-NMR** (126 MHz, DMSO-*d*₆, 25 °C): 169.70, 158.24, 148.96, 147.88, 144.09, 143.22, 141.19, 140.50, 139.10, 138.61, 129.05, 128.81, 128.45, 128.27, 128.17, 127.91, 127.82, 126.92, 126.50, 125.66, 123.81, 123.19, 119.05, 115.32, 114.12, 106.37, 103.76, 49.79, 49.42, 21.50, 20.38, 11.33, 10.83; **ES-MS** calculated: 545; found: 545 (M⁺-2H); **HRMS** calculated: 545,1824, found: 545,1823; **Elementary analysis:** calculated: C 58,75 %; H 4,63 %; found: C 59,12 %; H 4,87 %.

### Example 2

### Cytotoxicity (MTT) assay

Cells were seeded into 96-well microtitre plates to the expected target cell density (2,500-30,000 cells·well⁻¹, according to the particular cell line). The inoculant were pre-incubated to stabilize for 24 h (at 37°C and 5 % CO²). Fourfold dilutions (0-200 µmol·L⁻¹; in triplicates) of the tested compound **1** were added to the microtitre plate wells at time zero. The cells were treated with compound 1 for 72 h under standard cultivation conditions. Then, the cells were assayed using MTT for 4 h. Produced formazan, arisen by mitochondrial reduction of MTT, was dissolved in 10 % aqueous solution of sodium dodecyl sulphate (pH 5.5; 100 µL·well⁻¹, hole diameter is 6.54 mm) followed by overnight incubation. The produced formazan, originating from by mitochondrial reduction of MTT, was dissolved in 10 % aqueous solution of sodium dodecyl sulphate (pH 5.5; 100 µL·well⁻¹, hole diameter was 6.54 mm) followed by overnight incubation. The absorption of the produced formazan was measured at 540 nm using Labsystem iEMS Reader MF. The tumor cell inhibitory concentration (IC) of compound 1 was calculated using the following equation: IC = (OD_{drug} / mean OD_{control}) · 100 %. The IC₅₀ (Table 1) value (drug concentration being lethal for 50 % of the cells) was calculated from appropriate dose-response curves. *(*Noskova, V.; Dz ̌ubak, P.; Kuzmina, G.; Ludkova, A.; Stehlik, D.; Trojanec, R.; Janošt ̌akova, A.; Kor ̌inkova, G.; Mihal, V.; Hajduch, M. Neoplasma 2002, 49, 418-425*. 38.* Hajduch, M.; Mihal, V.; Minar ̌ik, J.; Faber, E.; Šafarova, M.; Weigl, E.; Antalek, P. Cytotechnology 1996, 19, 243-245*)*

### Example 3

Effect of salt 1 on tumor growth in vivo. Nu/nu mice bearing subcutaneously growing human breast carcinoma (MDA-MB-231) received salt 1 intravenously (5 mgkg-1, twice a week; ■). The control group (◆) consisted of untreated mice with a tumor. Statistically significant differences in the tumor volume of control mice versus mice treated with salt 1 are indicated (* p<0.05; ** p<0.01; n=5 mice per group).

### Industrial applicability

In the pharmaceutical industry for producing an agent for cancer treatment.

## Claims

1. A pentamethinium salt with an incorpoarted expanded quinoxaline unit of general formula I: 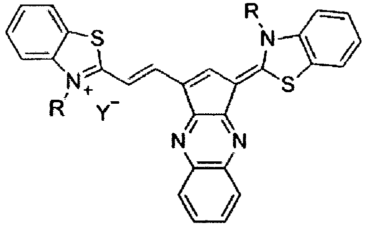 where group R is an alkyl chain having a length of C1 to C8, and Y is chloride, bromide or iodide.

2. Pentamethinium salt with an incorporated expanded quinoxaline unit according to claim 1, wherein R is selected from the group consisting of propyl and isopropyl.

3. A use of the pentamethinium salt with the incorpoarted expanded quinoxaline unit according claim 1 or 2 for the preparation of an agent with anticancer effects.

## Patentansprüche

1. Pentamethiniumsalz mit einer eingefügten erweiterten Chinoxalineinheit der allgemeinen Formel I: 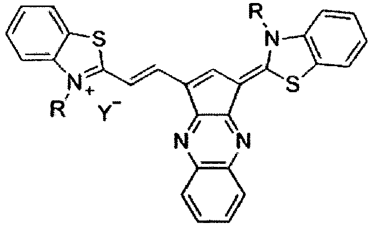 worin die Gruppe R eine Alkylgruppe mit einer Länge von C1 bis C8 und Y Chlorid, Bromid oder Iodid bedeutet.

2. Pentamethiniumsalz mit einer eingefügten erweiterten Chinoxalineinheit nach Anspruch 1, wobei R unter Propyl und Isopropyl ausgewählt ist.

3. Verwendung eines Pentamethiniumsalzes mit einer eingefügten erweiterten Chinoxalineinheit nach Anspruch 1 oder 2 für die Herstellung eines Wirkstoffes mit Anti-Krebs-Wirkungen.

## Revendications

1. Sel de pentaméthinium avec une unité de quinoxaline expansée incorporée de formule générale I : 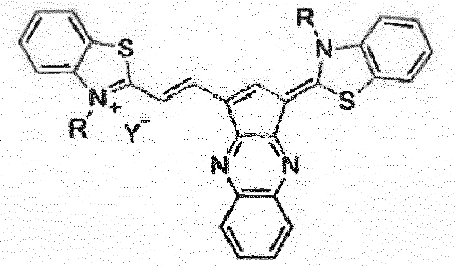 dans lequel le groupe R est une chaîne alkyle ayant une longueur de C1 à C8, et Y est du chlorure, du bromure ou de l'iodure.

2. Sel de pentaméthinium avec une unité de quinoxaline expansée incorporée selon la revendication 1, dans lequel R est choisi dans le groupe constitué par le propyle et l'isopropyle.

3. Utilisation du sel de pentaméthinium avec l'unité de quinoxaline expansée incorporée selon la revendication 1 ou 2 pour la préparation d'un agent ayant des effets anticancéreux.
